# EUROPEAN PATENT SPECIFICATION

(11) **EP 2 332 586 B1**
(45) Date of publication and mention of the grant of the patent: **30.10.2013**
(21) Application number: 10179856.9
(22) Date of filing: 26.11.2004
(51) Int. Cl.: A61L 26/00, A61K 9/70, A61L 15/58, A61L 15/60

(54) **Patch for treating Herpes labialis**
Klebepflaster fuer die Behandlung von Herpes labialis
Bande adhesive pour le traitement de Herpes labialis

(30) Priority: 28.11.2003 DK 200301761; 10.12.2003 US 528183 P
(43) Date of publication of application: 15.06.2011
(62) Divisional of application: 04797482.9
(73) Proprietor: Coloplast A/S, 3050 Humlebaek (DK)
(72) Inventor: Hansen, Grazyna, 2000, Frederiksberg C (DK); Nielsen, Anders Christian, 2880, Bagsvaerd (DK)

(56) References cited:
- EP-A- 0 591 898
- EP-A- 1 020 195
- JP-A- 2 250 824
- JP-A- 2 270 818
- JP-A- 6 142 178
- JP-A- 6 199 660
- JP-A- 9 124 462
- JP-A- 10 033 655

## Description

### BACKGROUND OF THE INVENTION

### 1. Field of the Invention

The present invention relates to adhesive patches, for use in the treatment of herpes as defined in claim 1.

### 2. Description of the Related Art

Conventionally, dressings or patches for the treatment or prevention of wounds or pressure sores or even unbroken skin comprise a backing layer and an adhesive layer. The adhesive layer is often bevelled at the edge portions in order to have a smooth appearance, and avoid wrinkling and focusing of stresses in the dressing often causing slipping of the adhesive and unintended detachment of the dressing. The central portion of the dressing may be quite thick in order to provide a cushioning effect and/or an absorbent effect. However, a thick dressing may be less flexible and less capable of following the movements of the skin, as well as it appears very visible at the skin.

It is often desired to have a dressing or patch, which is very discreet or even "invisible" when applied to the skin. This may be the case when the patch is worn in the face or on other exposed areas. The patch may be used for covering scratches, wounds, acne, scars or discoloured skin and may have to purposes: To camouflage irregularities at the skin and/or to treat the skin site, e.g. a wound or acne. Different product for these purposes is known. These are usually in the form of adhesive patches or dots to be applied on the desired body part. Most of them are rather thick and thus rather unflexible and often quite visible. Some may contain medicaments for topical delivery.

Us Patent No. 5,785,978 discloses an adhesive patch comprising active ingredients. The patch comprises an occlusive backing film and an adhesive layer. The shape of the patch is adapted to the body part on which they are applied. The patch is occlusive and thus not breathable and does not comprise any absorbent elements and may thus give rise to unwanted humidity under the patch resulting in irritation of the skin and reduced wear time.

Aside from camouflaging the treated site of the skin, it may, especially in the treatment of Herpes, pebbles or eczema, be desired to provide the site with an antimicrobial barrier rendering the site more hygienic and decrease the risk of contamination.

Treatment of Herpes may be exceptionally difficult by the use of adhesive patches due to the fact that they have to adhere partly or fully to the lips and are exposed to large movements/stretching, friction and high humidity, all factors that may reduce wear time substantially, as well as it may be desired that the patch is as discrete as possible.

None of the known products address the problems associated with the problem of producing an adhesive patch being capable of staying in place and blending into the skin in such a way that it may appear almost invisible.

It has now been found that the patch of the present invention fulfils the above-mentioned demands by providing an almost invisible patch with long wear time.

### SUMMARY OF THE INVENTION

The invention relates to a patch for use in treatment of Herpes Labialis, said patch comprising a backing layer and a layer of a skin-friendly adhesive for adhering to the skin or mucosa, said adhesive comprises hydrocolloid particles.

The invention provides a non-occlusive adhesive patch, i.e. one that will enable moisture on the surface of the skin to evaporate through the patch so as to prevent the undesired accumulation of moisture, which, if occurred, could cause the patch to detach or even facilitate the growth of bacteria beneath the patch.

Another object of the invention is to provide a lighter, more flexible and less obtrusive patch while still providing the excellent wear time and healing properties.

The patch of the invention is for application to the lip region.

The adhesive patch is ultra thin and capable of blending into the skin, rendering it extremely discreet for the user.

### Detailed Description of the Present Invention

The invention relates to a patch for use in treatment of Herpes Labialis, said patch comprising a backing layer, wherein the backing layer has a thickness of less than 30 µm, and a layer of a skin-friendly adhesive, said adhesive comprises hydrocolloid particles, wherein at least along the periphery of the patch the thickness of the adhesive layer is 20-300 µm and the vapour permeability of the patch is 200-1000 g/m2/24 h and the absorption of the patch is 40-600 g/m2/6h, and wherein the patch has a substantially uniform thickness.

The adhering surface preferably comprises hydrocolloid particles, the thickness of the adhesive being in the range of 20-300 µm, such as 25-300 µm, suchas 30-200 µm, such as 25-150 µm, such as 30-100 µm, and the vapour permeability of the dressing sheet being 200-1000 g/m², such as 300-800 g/m², such as 400-700 g/m², such as 450-650 g/m² measured over 24 hours. The patch has a substantially uniform thickness It has been found that a patch sheet with such thickness and vapour permeability provides a non-occlusive adhesive dressing sheet, i.e. one that enables moisture on, e.g., a skin surface to evaporate through the dressing sheet, so as to prevent undesired accumulation of moisture which could cause the dressing sheet to loose its adhering contact to the skin or promote bacterial growth between the sheet and the skin. Moreover, the low thickness of the dressing sheet results in a discrete appearance once applied to the application site.

The patch may preferably have an absorption of 50-400 g/m²/6h, more preferred 60-300 g/m²/6h and most preferred 70-250 g/m²/6h. The absorption is determined by immersing the patch in 0,9 M saline water at 37°C for 6 hours and then measuring the water uptake.

The presence of hydrocelloid in the adhesive provides a good environment for moist wound healing as well as for other skin conditions. By incorporating a limited amount of hydrocolloid in the adhesive, the patch is able to handle moisture in most conditions.

The present invention discloses a patch with a unique combination of a limited absorption combined with a high permeability. This combination provides optimal conditions for moist wound healing and long wear time when applied to lips.

Patches with very high permeability are known in the market, examples are SafeTac from Mölnlycke and Tielle from Johnson & Johnson. But these patches are not capable of providing moist wound healing. Products with a high absorption are also known. Examples of such are traditional hydrocolloid dressings. But these dressings may have a very low permeability until they are fully satisfied with moisture and then the permeability will rise, but due to the gelling of the adhesive the adhesive tack of the dressing will be reduced, and the dressing may delaminate from the skin. Furthermore, these dressings are rather thick and unflexible and thus unsuitable for treatment of Herpes.

For application to the lip region, the wear time is preferably at least 2 hours, more preferred at least 3 hours and most preferred at least 4 hours.

Due to the high permeability of the patch of the present invention the patch may not need to be able to store large amounts of exudates/moisture, but due to evaporation the overall moisture handling capacity will be high, hence the flux of moisture through the patch is high.

The thickness of the dressing is often chosen from the amount of exudates expected from the wound, i.e. a thick dressing for a highly exuding wound. The idea is the thicker dressing, the more hydrocolloid particles and the higher absorption. However, a thick dressing may have a lower permeability. By using a thin patch with a high permeability, and due to the thickness, restricted amount of hydrocolloid particles, the moisture will be absorbed by the particles, thus not macerating the wound, and then the moisture will be released again through the top layer during evaporation. In this way the patch will be able to handle much higher amounts of moisture than the absorbency of the hydrocolloid particles.

Very thin dressings or patches are usually prepared with non-absorbent adhesives, such as polyacrylates. The presence of hydrocolloid particles, however, provides a moist wound healing environment by absorbing moisture, thus leaving the wound or skin neither too dry nor too wet. The absence of absorbent particles may give rise to maceration of the skin or drying out of a wound.

Though the term adhesive is used herein it is understood that the term may cover any substance having adherent properties, such as adhesives, silicone or rubbery substances, petrolatum or the like, and hydrocolloid adhesives. The adhesive may be a pressure sensitive adhesive of any suitable kind known per se.

The thickness of the adhesive layer of the patch of the present invention is substantially constant over the surface.

It is preferred that the thickness of the adhesive layer is 20-300 µm, more preferred 25-150 µm and most preferred 30-100 µm and even most preferred 50-80 µm.

The patch has a substantially uniform thickness. Due to the low thickness, bevelling may not be necessary in order to ensure good tack and reduce rolling up of the edge portions.

Also described is a patch that is 100-200 µm thick. The obtained patch is thus thick enough to be handled without folding or wrinkling but at the same time remarkably thinner than traditional hydrocolloid dressings.

A thin layer of adhesive is desired, as this will reduce the overall thickness of the patch. The thinner the dressing or patch is, the more flexible and more capable of following the movements of the body it is and the more discrete it appears.

The patch may work like a second skin and easily follow the movements of the underlying skin.

The surface area of the dressing sheet may e.g. be less than 5 cm², such as at most 4 cm², such as at most 2 cm², such as in the range of 1-2 cm², or smaller, such as 0.08 - 1 cm², such as 0.1 - 0.8 cm², such as 0.12 - 5 cm². For facial application, e.g. of a thin film patch, the surface area is usually less than 5 cm².

The vapour permeability of the patch is preferably 300-1100 g/m², more preferred 400-850 g/m², and most preferred 450-750 g/m².

The patch may have an optimal balance between a limited absorption and an appropriate permeability. Together these properties provides a patch with excellent moisture handling qualities compared to common thin dressings which may have a thicker layer of adhesive and/or a non-absorbing adhesive.

The adhesive of the patch of the invention may be any suitable skin-friendly adhesive. The adhesive further comprises particles of hydrocolloids and may comprise super absorbing particles or fibres.

The skin-friendly adhesive may be any skin-friendly adhesive known per se for production of medical articles, which are to be adhered to human skin. The adhesive may suitably be of the type disclosed in US patent Nos. 4,231,369, 4,367,732, 4,867,748, and 5,714,225. Especially preferred are the adhesives disclosed in US patent Nos. 4,367,732, and 5,714,225.

The patch of the present invention may in one embodiment of the invention be in the form of a mono-phase adhesive, i.e. made from one adhesive component or in accordance with another embodiment of the invention be in the form of a two-zone adhesive, e.g. of the general type disclosed in US patent No. 5,714,225, i.e. a part of or all of the adhesive areas of the patch having maximum thickness being constituted by more than one type of adhesive.

The particle size of the hydrocolloid particles influence on the thickness of the adhesive layer, as it is difficult to prepare an adhesive layer being thinner than the size of the hydrocolloid particles.

The physical form of conventional hydrocolloids is relative coarse and irregular particles, typically about 60-100 µm and the particles are in the form of a dry powder. In order to obtain finer particles, the hydrocolloid may be milled and/or sifted.

Suitable hydrocolloids for the patch of the present invention include synthetic polymers prepared from single or multiple monomers, naturally occurring hydrophilic polymers or chemically modified naturally occurring hydrophilic polymers. The hydrocolloid polymers may be linear or cross-linked. This include natural or chemically modified natural polymers like cellulosics such as CMC, chitosan, pectin, guar gum, starches or dextrines, collagenes and gelatine and synthetic polymers like polyacrylic acid, polyvinylealcohol/acetate, polyhydroxyalkyl acrylates and methacrylates, polyacrylamides, polystyrene sulfonates, polyvinyl pyrilidone, polyglycols, copolymers, grafts of such, copolymers or compositions of such.

In a preferred embodiment of the invention the hydrocolloid particles have an average size being substantially less than 125µm, more preferred less than 100 µm, even more preferred less than 75 µm and most preferred less than 50 µm.

The adhesive layer may be in the form of a pattern, such as geometric pattern or a random pattern, or the adhesive layer may comprise larger interruptions in the form of areas with no adhesive, e.g. the central part of the patch.

It is preferred that the adhesive layer is uninterrupted. The uninterrupted layer provides several advantages, such as less wrinkling, better invisibility and better blending into the skin. Backing layer being uncoated with adhesive may be more non-transparent and thus more visible. Furthermore, the pattern may leave marks on the skin when the patch is removed.

The adhesive patch may be in a flat continuous layer from 20 g/m² up to 1000 g/m².

The patch is for treatment of herpes and the patch is applied to the lip or lip region, being discrete and without causing discomfort.

It is suitable that the backing layer is a substantially water-impervious film which protects the adhesive from being adversely affected when the wearer is bathing or in case of incidental wetting of the area and especially when the adhesive is water absorbing.

The backing layer may be any water impervious layer or film may be of any suitable material known per se for use in the preparation of wound dressings e.g. a foam, a non-woven layer or a polyurethane, polyethylene, polyester or polyamide film. In accordance with the invention it has been found in practice that the use of a thinner backing layer or film than is normally used when preparing medical dressings, an improved stretchability and adaptability is obtained at the same time as the modulus is reduced.

The backing layer may preferably be an elastic, flexible and non-sticking film that protects the adhesive during storage as well as during use.

The water impervious, but vapour permeable layer or film is preferably a low-friction flexible polymer film reducing the risk of unwanted stress in the area of application.

An especially suitable material for use as a water impervious film is a polyurethane film. A preferred low friction film material is disclosed in US patent No. 5,643,187.

The backing layer may have a suitable thickness for the intended use. If the patch were desired for an "invisible" face patch, a rather thin film would be appropriate. The backing layer has a thickness of less than 30 µm.

A preferred thickness of this film may be below 20 microns, more preferred about 12-18 microns, e.g. about 15 microns, thus resulting in a significant decrease of the modulus, compared to a film that is normally used when preparing medical dressings. An improved stretchability and adaptability is obtained at the same time as the modulus is reduced.

In accordance with a preferred embodiment the backing layer is a film showing a low surface friction. Furthermore, the surface may be opaque, with a reflection being near to the reflection of skin, thus enabling the patch to blend with the skin colour and reflection and be less visible.

The backing layer may be coloured in suitable colours, e.g. flesh-colour or it may carry ornamentals. The backing layer may be transparent, translucent, opaque or non-transparent, depending on the intended use.

In order to be able to visually blend into the skin and become invisible, it is desired that the patch have a reflectance being close to that of the skin. It is preferred that the reflectance is lower than 5, more preferred between 4,5 and 1, and even more preferred between 4 and 1,5. The reflectance is measured on a Micro-TRI-gloss apparatus from BYK-Gardner and is measured with reference to an ASTM D523 standard. The measuring angle is 60°. The higher value of reflectance, the higher is the gloss of the product.

A patch of the invention may preferably be sterilised for avoiding the risk of causing infections when applied to skin areas having broken skin.

It is not critical whether or not the patch is sterilised, if the patch is applied to non-broken skin.

The patch of the invention is optionally covered in part or fully by one or more release liners or cover films to be removed before or during application.

A protective cover or release liner may for instance be siliconized paper. It does not need to have the same contour as the patch, e.g. a number of patches may be attached to a larger sheet of protective cover. The protective cover is not present during the use of the patch of the invention and is therefore not an essential part of the invention.

Furthermore, the patch of the invention may comprise one or more "non touch" grip(s) known per se for applying the patch to the skin without touching the adhesive layer. Such a non-touch grip is not present after application of the patch. For larger patches it is suitable to have 2 or 3 or even 4 "non-touch" grips.

The patch of the invention may further comprise one or more cover layers. The cover layer may protect the patch during storage and help easy application of the patch. The cover layer is removed during or after application.

Preferably, in all embodiments of the present invention, the patch is provided in the form of a backing layer with an adhesive applied to one surface thereof. The adhering surface of the patch may comprise a pharmaceutically active substance.

Due to its discrete appearance and the easy applicability provided by the carrier system, the patch of the invention is used for the treatment of herpes, and may be advantageously used with medicaments known per se for such purposes being contained in the adhesive or being applied thereto. Suitable anti viral medicaments for the treatment of herpes may for example comprise aciclovir or penciclovir.

The above mentioned pharmaceutically active substances may be applied to the adhering surface of the dressing sheet after completion of the adhering coating, or they may be mixed into the adhesive prior to coating thereof onto the backing layer.

In one embodiment of the invention the medicament may be applied to the patch before application. An amount of a gel or cream may be applied to the central part of the patch before application to the treatment site. In the treatment of Herpes it may be advantageous to apply an Acyclovir containing cream or gel such a Zovir before application to the Herpes site.

The patch may comprise one or more cavities for accommodating a medicament. The cavities may be in the form of a dome shaped portion or an indentation.

### EXAMPLES

### EXAMPLE 1

The reflectance of a patch according to the invention has been measured. The test has been carried out using a Micro-TRI-gloss apparatus from BYK-Gardner. The reflectance is measured with reference to an ASTM D523 standard. The angle was 60°. The higher value, the higher gloss of the product.

4 measurements on 3 persons were obtained, testing skin, a patch according to the present invention and a competitor product "T-zone". The T-zone patch is from Brodie & Stone plc and is a product for treatment of acne and comprises a top film coated with polyacrylate adhesive. The product further comprises an antiseptic agent in the form of Tea tree oil. The results of the reflectance est are shown in Table 1 below.

As can be seen from the results, the reflection of the patch of the present invention is very close to the reflection of the skin, and thus contributes to the invisibility of the patch. The competitor product, T-zone, has much higher reflectance, and is thus more visible, and would not be suitable for a discrete appearance.

**TABLE 1**

| | **Skin** | **Patch** | **T-zone** |
|---|---|---|---|
| **Person 1** | | | |
| #1 | 2,6 | 2,8 | 21,4 |
| #2 | 2,6 | 3,1 | 18,2 |
| #3 | 2,6 | 2,9 | 26,6 |
| #4 | 3,2 | 2,3 | 16,9 |

| **Person 2** | | | |
|---|---|---|---|
| #1 | 2,1 | 2,7, | 10,0 |
| #2 | 1,8 | 3,6 | 6,2 |
| #3 | 2,3 | 2,2 | 6,0 |
| #4 | 2,4 | 3,3 | 5,0 |

| **Person 3** | | | |
|---|---|---|---|
| #1 | 2,7 | 4,2 | 10,3 |
| #2 | 2,8 | 4,4 | 9,6 |
| #3 | 2,7 | 3,2 | 21,2 |
| #4 | 3,0 | 3,5 | 20,7 |

### EXAMPLE 2

A clinical investigation has been carried out at Bispebjerg Hospital, Department of Dermato-venerology and Wound Healing Center. 85 persons with Herpes Labialis completed the study. The patients were provided with patches of the present invention during an outbreak of the virus and filled in a questionnaire. Results from the questionnaire are shown below.

94% answered that the patch was extremely or very elastic/flexible during the outbreak (followed the movements of the skin).
91% answered that the patch was extremely pleasant to wear during the outbreak.
85% said that the patch relieved the outbreak.
88% said that the patch made the outbreak less visible.
81 % said that the cold sore did not form a crust, and among these found 86% that this was an advantage.
79% would be interested to buy the patches if they were obtainable from pharmacies/drug stores.
87% found that the patches made the outbreak easier to cope with / live with compared to traditional treatment.
65% found that the patches decreased the healing time of the outbreak.

It is concluded from the study that the patch of the present invention provides reduced healing time, and higher comfort during the outbreak, compared to traditional Herpes treatment.

### EXAMPLE 3

A comparison between treatment of Herpes Labialis with an Acyclovir containing cream and treatment with a patch of the present invention. The study was performed by Bispebjerg Hospital, Department of Dermato-venerology and wound Healing Center. The results are shown in Table 2.

**TABLE 2**

| | Treatment with cream | Treatment with patch |
|---|---|---|
| Redness/swollenness of skin in area of herpes | yes | yes |
| Blister formation | yes | only small blisters |
| Moist ulceration | yes | less ulceration |
| Formation of crust | yes | no |
| Secondary infection | maybe | no |

The treatment with the patch of the present invention moderates the formation of blisters and prevents the formation of a crust. The absence of crust seems to reduce the healing time and cause less inconvenience for the patient. Secondary infections are avoided, which also contributes to reduce healing time.

## Claims

1. A patch for use in treatment of Herpes Labialis, said patch comprising a backing layer, wherein the backing layer has a thickness of less than 30 µm, and a layer of a skin-friendly adhesive, said adhesive comprises hydrocolloid particles, wherein at least along the periphery of the patch the thickness of the adhesive layer is 20-300 µm and the vapour permeability of the patch is 200-1000 g/m2/24 h and the absorption of the patch is 40-600 g/m2/6h, and wherein the patch has a substantially uniform thickness.

2. A patch according to any of the preceding claims, wherein the absorption of the patch is 50-400 g/m²/6h.

3. A patch according to any of the preceding claims, wherein the thickness of the adhesive layer is 30-200 µm.

4. A patch according to any of the preceding claims, wherein the vapour permeability of the patch is 300-800 g/m².

5. A patch according to any of the preceding claims, wherein the hydrocolloid particles have a size being substantially less than 125 µm.

6. A patch according to any of the preceding claims, wherein the hydrocolloid particles have a size being substantially less than 100 µm.

7. A patch according to any of the preceding claims, wherein the patch has a reflectance lower than 5.

8. A patch according to any of the preceding claims, wherein the backing layer is a polyurethane film.

9. A patch according to any of the preceding claims, wherein the adhesive layer is uninterrupted.

10. A patch according to any of the preceding claims, wherein the patch comprises an absorbent pad.

11. A patch according to any of the preceding claims, wherein the patch comprises one or more cavities.

12. A patch according to any of the preceding claims, wherein the thickness of the backing layer is less than 20 µm.

13. A patch according to any of the preceding claims, wherein the patch has a surface area of 5 cm² or less.

14. A patch according to any of the preceding claims, wherein the patch further comprises one or more active ingredients.

15. A patch according to claim 14, wherein the active ingredient is mixed into the adhesive.

16. A patch according to claim 14-15, wherein the active ingredient is an anti viral medicament.

17. A patch according to claim 16, wherein the anti viral medicament comprises aciclovir or penciclovir.

## Patentansprüche

1. Pflaster zur Verwendung bei der Behandlung von Herpes labialis, umfassend eine Rückschicht, wobei die Rückschicht eine Dicke von weniger als 30 µm aufweist, und eine Schicht eines hautfreundlichen Klebstoffs, wobei der Klebstoff Hydrokolloidteilchen umfasst, wobei die Dicke der Klebstoffschicht zumindest entlang der Peripherie des Pflasters 20-300 µm beträgt und die Wasserdampfdurchlässigkeit des Pflasters 200-1000 g/m²/24 h beträgt und die Absorption des Pflasters 40-600 g/m²/6 h beträgt, wobei das Pflaster eine weitgehend einheitliche Dicke aufweist.

2. Pflaster nach dem vorhergehenden Anspruch, wobei die Absorption des Pflasters 50-400 g/m²/6 h beträgt.

3. Pflaster nach einem der vorhergehenden Ansprüche, wobei die Dicke der Klebstoffschicht 30-200 µm beträgt.

4. Pflaster nach einem der vorhergehenden Ansprüche, wobei die Wasserdampfdurchlässigkeit des Pflasters 300-800 g/m² beträgt.

5. Pflaster nach einem der vorhergehenden Ansprüche, wobei die Hydrokolloidteilchen eine Größe aufweisen, die weitgehend weniger als 125 µm beträgt.

6. Pflaster nach einem der vorhergehenden Ansprüche, wobei die Hydrokolloidteilchen eine Größe aufweisen, die weitgehend weniger als 100 µm beträgt.

7. Pflaster nach einem der vorhergehenden Ansprüche, wobei das Pflaster einen Reflexionsgrad von weniger als 5 aufweist.

8. Pflaster nach einem der vorhergehenden Ansprüche, wobei es sich bei der Rückschicht um eine Polyurethanfolie handelt.

9. Pflaster nach einem der vorhergehenden Ansprüche, wobei die Klebstoffschicht ununterbrochen ist.

10. Pflaster nach einem der vorhergehenden Ansprüche, wobei das Pflaster ein Saugkissen umfasst.

11. Pflaster nach einem der vorhergehenden Ansprüche, wobei das Pflaster einen oder mehrere Hohlräume umfasst.

12. Pflaster nach einem der vorhergehenden Ansprüche, wobei die Dicke der Rückschicht weniger als 20 µm beträgt.

13. Pflaster nach einem der vorhergehenden Ansprüche, wobei das Pflaster eine Oberfläche von 5 cm² oder weniger aufweist.

14. Pflaster nach einem der vorhergehenden Ansprüche, wobei das Pflaster ferner einen oder mehrere Wirkstoffe umfasst.

15. Pflaster nach Anspruch 14, wobei der Wirkstoff in den Klebstoff eingemischt ist.

16. Pflaster nach Anspruch 14-15, wobei es sich bei dem Wirkstoff um ein antivirales Medikament handelt.

17. Pflaster nach Anspruch 16, wobei das antivirale Medikament Aciclovir oder Penciclovir umfasst.

## Revendications

1. Patch pour utilisation dans le traitement de l'herpès labial, ledit patch comprenant une couche de support, la couche de support ayant une épaisseur inférieure à 30 µm, et un adhésif sans danger pour la peau, ledit adhésif comprenant des particules d'hydrocolloïde, dans lequel, au moins le long de la périphérie du patch, l'épaisseur de la couche d'adhésif est de 20 à 300 µm et la perméabilité à la vapeur du patch est de 200 à 1000 g/m²/24h et l'absorption du patch est de 40 à 600 g/m²/6h, et le patch ayant une épaisseur sensiblement uniforme.

2. Patch selon la revendication précédente, l'absorption du patch étant de 50 à 400 g/m²/6h.

3. Patch selon l'une quelconque des revendications précédentes, l'épaisseur de la couche d'adhésif étant de 30 à 200 µm.

4. Patch selon l'une quelconque des revendications précédentes, la perméabilité à la vapeur du patch étant de 300 à 800 g/m².

5. Patch selon l'une quelconque des revendications précédentes, les particules d'hydrocolloïde ayant une taille qui est sensiblement inférieure à 125 µm.

6. Patch selon l'une quelconque des revendications précédentes, les particules d'hydrocolloïde ayant une taille qui est sensiblement inférieure à 100 µm.

7. Patch selon l'une quelconque des revendications précédentes, le patch ayant une réflectance inférieure à 5.

8. Patch selon l'une quelconque des revendications précédentes, la couche de support étant un film de polyuréthane.

9. Patch selon l'une quelconque des revendications précédentes, la couche d'adhésif étant ininterrompue.

10. Patch selon l'une quelconque des revendications précédentes, le patch comprenant un tampon absorbant.

11. Patch selon l'une quelconque des revendications précédentes, le patch comprenant une ou plusieurs cavités.

12. Patch selon l'une quelconque des revendications précédentes, l'épaisseur de la couche de support étant inférieure à 20 µm.

13. Patch selon l'une quelconque des revendications précédentes, le patch ayant une surface de 5 cm² ou moins.

14. Patch selon l'une quelconque des revendications précédentes, le patch comprenant en outre une ou plusieurs substances actives.

15. Patch selon la revendication 14, la substance active étant mélangée dans l'adhésif.

16. Patch selon la revendication 14 à 15, la substance active étant un médicament antiviral.

17. Patch selon la revendication 16, le médicament antiviral comprenant l'aciclovir ou le penciclovir.
